# EUROPEAN PATENT SPECIFICATION

(11) **EP 2 609 860 B1**
(45) Date of publication and mention of the grant of the patent: **26.07.2017**
(21) Application number: 12199640.9
(22) Date of filing: 28.12.2012
(51) Int. Cl.: A61B 6/00, A61B 6/14

(54) **Apparatus and method for dental radiography with improved accuracy**
Vorrichtung und Verfahren zur Zahnröntgenaufnahme mit verbesserter Genauigkeit
Appareil et procédé de radiographie dentaire avec une précision améliorée

(30) Priority: 28.12.2011 IT BO20110765
(43) Date of publication of application: 03.07.2013
(73) Proprietor: CEFLA S.C., 40026 Imola (IT)
(72) Inventor: Bianconi, Davide, 40023 Castel Guelfo di Bologna (IT); Langella, Luciano, 22070 Casnate (IT); Molteni, Roberto, 37013 Caprino Veronese (IT); Righini, Dario, 40026 Imola (IT)
(74) Representative: Herrmann, Franz

(56) References cited:
- EP-A1- 1 697 794
- EP-A1- 2 198 783

## Description

The invention relates to an apparatus for dental radiography comprising:
- an imaging system comprising at least one X-ray detector having an X-ray sensitive area, the imaging system further comprising an X-ray source having a focal spot, which emits an X-ray beam having an optical axis which intersects with the X-ray sensitive area of the X-ray detector,
- a support holding the imaging system and arranged for performing a trajectory of the imaging system for scanning a patient, wherein the trajectory of the imaging system comprises a pivotation of the X-ray source around a mechanical rotation axis and a translation of the mechanical rotation axis.

The invention further relates to a method for dental radiography.

Such an apparatus and such a method is known from US 2004/0190678 A1. The known apparatus is provided with a support having a base attached to the ground. Mounted on the base is a frame which can slide along the base in the vertical direction. Attached to the frame is a horizontal extension which is provided with a driving mechanism for a rotary unit. On one end, the rotary unit is holding an X-ray source and on the other end, the rotary unit is provided with an X-ray detector. The rotary arm can perform a rotation around a rotation axis for taking panoramic images of the dentition of a patient.

Besides panoramic images, the known apparatus can also be used for cephalography. For this purpose, one end of a further extension is attached to the slidable frame. The other end of the further extension is provided with a detector for cephalography. In the cephalographic mode, the rotary arm performs a pivotation around the rotation axis such that a sweeping X-ray beam is generated, which impinges on the detector provided for cephalography. The detector for cephalography can also be moved to keep the detector in the sweeping X-ray beam.

The beam emitted by the X-ray source is a fan-shaped beam, which extends along a vertical plane. The magnification in the vertical direction of an object scanned by the fan-shaped beam is determined by the ratio of the distance between the focal spot and the detector and the distance between the focal spot and an object plane. For pivoting the fan-shaped beam in horizontal direction, the fan-shaped X-ray beam is rotated around the vertically aligned rotation axis. If the rays of the X-ray beam are projected on a horizontal plane at various instants of time during the scanning process, the rays intersect at the location of the rotation axis. Therefore, the X-rays seem to originate from a virtual projection center at the location of the rotation axis. In consequence, the horizontal magnification is determined by the ratio of the distance between the rotation axis and detector and the distance between the rotation axis and the object. Thus, the vertical and horizontal magnifications are different.

EP 1 259 162 B1 proposes to shift the virtual projection center formed by the rotation axis to the focal spot of the X-ray source if cephalographic images are taken. The projection center in the horizontal is consequently the actual projection center, namely the focal spot of the X-ray source. Thus the vertical and horizontal magnification can be made equal.

EP 1 697 794 B1 proposes the same for panoramic imaging.

EP 2 198 783 discloses a panoramic and cephalography dental imaging apparatus with a shiftable source. Panoramic and cephalographic images are generally used for planning orthodontic measures. It is therefore important that these images reflect the actual geometry of the dentition as accurately as possible.

Proceeding from this related art, the present invention seeks to provide an apparatus for dental radiography with improved accuracy.

This object is achieved by an apparatus having the features of the independent claim. Advantageous embodiments and refinements are specified in claims dependent thereon.

In the apparatus according to the invention, the combined pivotation and translation results in an effective pivotation of the X-ray source around a virtual projection center of the X-ray beam, wherein the virtual projection center is located along the optical axis behind the focal spot of the X-ray source as seen from the X-ray detector.

Such an arrangement enables to put the virtual projection center at such a large distance from the object, that the geometry of the beam equals or at least approaches the geometry of a beam with a projection center at infinity, that is a beam with parallel rays. Such a beam creates an accurate projection of the dentition without any distortion which would occur, if the projection center were in proximity of the object so that different object planes were magnified differently.

In one particular embodiment, the rotation axis is aligned in the vertical direction and the translation of the rotation axis is performed along a horizontal plane. The horizontal magnification which is particularly important for getting an accurate image of the dentition of a patient can thus be kept uniform over the head of the patient resulting in low distortion.

In another particular embodiment, the at least one X-ray detector is a digital line detector and the X-ray beam is fan-shaped. A linear detector is particular suitable for panoramic imaging since the position of the linear detector may change continuously while a panoramic images is taken. For cephalography, the linear detector and the fan-shaped beam are generally moved in a direction at right angle with respect to the longitudinal axis of the detector or the plane of the beam, in order to obtain two-dimensional images.
The longitudinal axis of the digital line detector is usually aligned in the vertical direction and the fan-shaped X-ray beam usually extends along a vertical plane. If the rotation axis is vertically aligned, two-dimensional images can be taken by pivoting the fan-shaped beam around the rotation axis and by moving the X-ray detector such, that X-ray detector and X-ray beam coincide. The distance between the virtual projection center and the focal spot is greater than the distance between the focal spot and the detector. Thus, the beam angle of the X-ray beam can be considerable reduced resulting in a more uniform magnification of various object planes.
For obtaining geometry similar to the geometry of devices for clinical dental radiography, the distance between the virtual projection center and the focal spot ranges between 3 meter and 5 meter.
In one particular embodiment, the support comprises an extension arm and a rotary arm attached to the extension arm, which comprises a driving mechanism arranged for pivoting the rotary arm around the rotation axis and for translating the rotation axis. In this particular configuration, the X-ray source can be mounted on the rotary arm. This is a typical configuration of a machine, which is used in medical offices of local dentists.

For allowing an appropriate setup of the X-ray beam for various imaging modes, the X-ray source and/or the at least one X-ray detector can be arranged for performing a relative motion with respect to the rotary arm.

In a typical configuration, the X-ray source is mounted on one end of the rotary arm and an X-ray detector for panoramic imaging is mounted on the other end of the rotary arm.

The support can further comprise a further extension for holding a detector for cephalography, wherein the detector for cephalography is movable for keeping the detector for cephalography in coincidence with the sweeping X-ray beam.

In one particular embodiment, a single detector is used for panoramic imaging and cephalography. The single detector ought to be manually mountable and detachable on the rotary arm and on the further extension for cephalography.

The apparatus generally also comprises a control unit arranged for controlling the trajectories of the imaging system in a panoramic operation mode and for controlling the trajectories of the imaging system in a cephalographic operation mode.

Further advantages and properties of the present invention are disclosed in the following description, in which exemplary embodiments of the present invention are explained in detail based on the drawings:
- Figure 1: is a top view of an apparatus for dental radiography in the panoramic operation mode;
- Figure 2: is a top view of the apparatus from Figure 1 in the cephalographic operation mode;
- Figure 3: is a side view of the apparatus from Figure 1;
- Figure 4: is a schematic top view of the beam in a configuration, in which, in a projection on a horizontal plane, a focal spot of an X-ray source is the virtual projection center of the beam;
- Figure 5: is a schematic side view of the beam in a configuration, in which, in a projection on a horizontal plane, the focal spot of the X-ray source is the virtual projection center of the beam;
- Figure 6: is a schematic top view of the beam in an ideal configuration, in which, in a projection on a horizontal plane, the virtual projection center is at infinity;
- Figure 7: is a schematic side view of the beam in an ideal configuration, in which, in a projection on a horizontal plane, the virtual projection center is at infinity;
- Figure 8: is a schematic top view of the beam in a configuration, in which, in a projection on a horizontal plane, the virtual projection center is behind the focal spot of the beam; and
- Figure 9: is a schematic side view of the beam in a configuration, in which, in a projection on a horizontal plane, the virtual projection center is behind the focal spot of the beam.

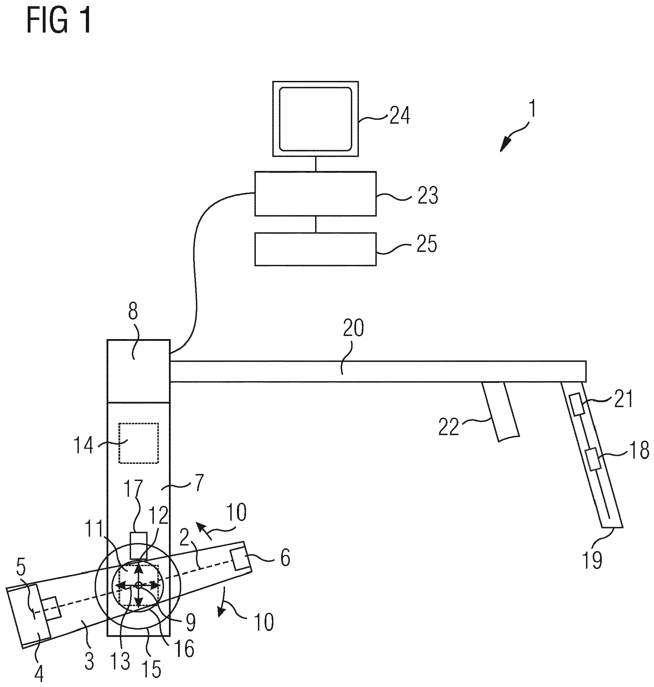

Figure 1 shows a view from above on an apparatus 1 for X-ray dental radiography, in particular for dental cephalography, panoramic imaging and optionally also for tomographic imaging. The apparatus 1 is preferably a horizontal scanning system, using an X-ray beam 2 which has the shape of a fan which generally extends along a vertical aligned plane.

The imaging system of the apparatus 1 comprises a rotary arm 3. One end of the rotary arm 3 an X-ray source 4 is accommodated, which comprises a focal spot 5 from which the fan-shaped X-ray beam 2 originates. The focal spot 5 is generally formed by the anode of the X-ray source 4, which may be a fixed or rotating anode.

On the other end of the rotary arm 3, a panoramic X-ray detector 6 is disposed. The panoramic detector 6 is generally a digital line detector having a single column of detector pixels aligned in the vertical direction. The X-ray detector 6 may be a CCD sensor operated in TDI mode or a CMOS sensor operated in frame acquisition mode or any other suitable digital X-ray detector.

The rotary arm 3 is pivotably mounted on a horizontal extension 7 that is attached to a column 8. The rotary arm 3 can be pivoted in a rotational plane around a rotation axis 9 that defines the mechanical rotation center of a rotational movement 10 of the rotary arm 3 within a rotational plane extended by the rotational movement of the rotary arm 3. The rotational motion 10 of the rotary arm 3 is driven by a cinematic unit 11. The cinematic unit 11 also allows for displacing the rotation axis 9 along two linear independent axes 12 and 13 aligned along the rotational plane. The rotational axis 9 can thus be moved with two degrees of freedom within a plane extending along the rotational plane in which the rotation of the rotary arm 3 occurs. The cinematic unit 11 is preferably provided with multi-axis independent actuators and might be a XY linear table equipped with a rotational actuator.

In most cases, the rotation axis 9 will be vertically aligned so that the rotational plane is a horizontal plane. In consequence also the translational movements of the rotation axis 9 occur in horizontal directions.

The operation of the cinematic unit 11 is controlled by a control unit 14 which is capable of independently driving each axis 12 and 13 of the cinematic unit 11. Thus, a translation of the mechanical rotation center of the rotary arm 3 in the useful range of the XY table and a rotational motion of the whole rotary arm 3 can be performed.

During a panoramic scanning process, a patient 15, in particular a head 16 of the patient 15 is irradiated by the X-ray beam 2. The head 16 of the patient 15 is held in a stationary position with respect to the apparatus 1 by a positioning device 17, such as a bite, on which the patient 15 has to bite during the panoramic scanning process. In consequence, the spatial relation between the X-ray source 4, the head 16 and the X-ray detector 6 is known at any time during the panoramic scanning process.

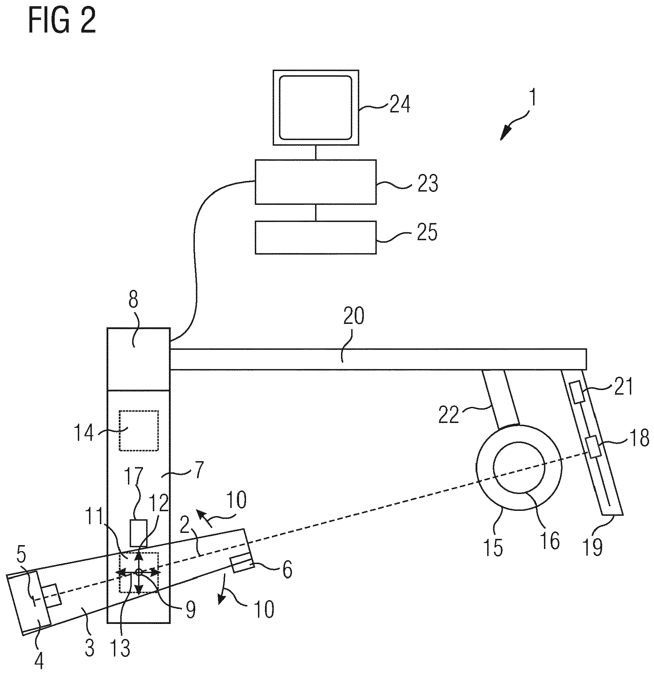

The imaging system of the apparatus 1 further comprises a cephalographic X-ray detector 18 mounted on a detector arm 19. The X-ray detector 18 may be a CCD sensor operated in TDI mode or a CMOS sensor operated in frame acquisition mode or any other suitable digital detector. The detector arm 19 is connected to the column 8 by a connecting strut 20. The X-ray detector 18 is capable of performing a linear translation in a direction extending along the rotational plane, in which the rotation of the rotary arm 3 occurs. In the embodiment shown in Figure 1 and 2, the linear translation of the X-ray detector 18 can be synchronized with the motion of the rotary arm 3, so that the cephalographic X-ray detector 18 is always kept in the X-ray beam 2 during cephalographic scanning. The X-ray detector 18 is actuated by a linear drive 21, which may also be controlled by the control unit 14. Thus the control unit 14 controls the roto-translation of the rotary arm 3 and the linear translation of the X-ray detector 18. The control unit 14 further controls the synchronization of both motions. Thus, the control unit 14 ensures that the fan-shaped X-ray beam 2 always impinges perfectly on the X-ray detector 18 during the cephalographic scanning process.

As can be recognized from Figure 2, in the cephalographic operation mode, the patient 15, in particular the head 16 of the patient 15 is irradiated by the X-ray beam 2. For this purpose, the panoramic detector 6 can be moved out of the beam, so that the beam 2 can impinge on the cephalographic detector 18. The head 16 of the patient 15 is held in a stationary position with respect to the apparatus 1 by a positioning device 22, such as a cephalostat. In consequence, the spatial relation between the X-ray source 4, the head 16 and the X-ray detector 18 is known at any time during the scanning process.

The apparatus 1 is operated in the cephalographic operation mode as follows: After the patient 15 has been positioned with respect to the apparatus 1 by the positioning device 22, the rotary arm 3 is pivoted around the rotation axis 9. While the X-ray beam 2 scans the head 16 of the patient 15, the X-ray detector 18 is read out at a predefined clock frequency, in synchronization with the linear translation of the second driving system. The image data read out from the X-ray detector 18 are then used for reconstructing a radiographic image of the head 16 of the patient 15.

The apparatus 1 is further provided with an image processing device 23. The image processing device 23 is arranged for processing the radiographic images generated by the detector 6 and 18. In particular, image data generated during both scanning processes can be used for generating a well-known panoramic view of the dentition of the patient, if the apparatus is operated in the panoramic mode, or anterioposterior views or lateral views of the head 16 of the patient 15, if the apparatus is operated in the cephalographic mode. These views can be displayed on a display 24 that is connected to the image processing device 23. The image processing device 23 can finally also be connected to an input device 25. The image processing device 23 can be a personal computer, the display device 24 can be a screen and the input device 25 a keyboard, a mouse or a similar device.

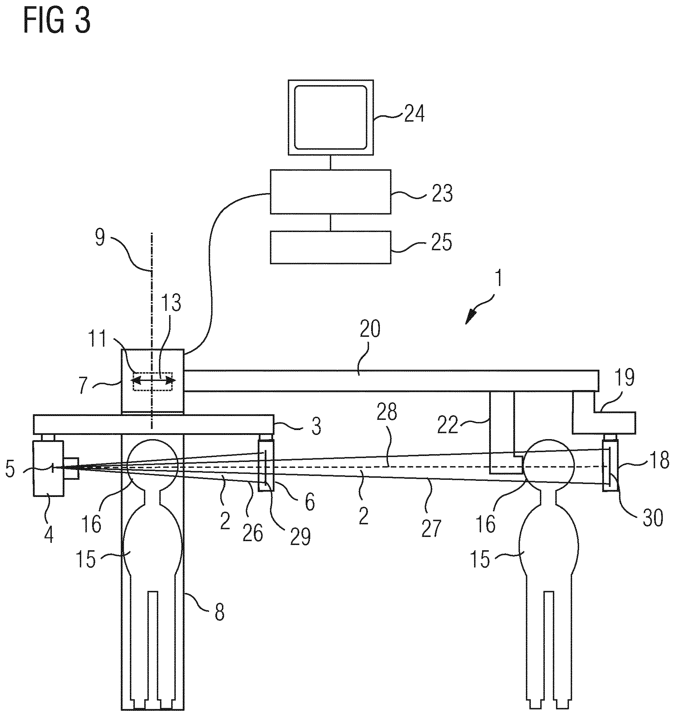

Figure 3 shows a side view of the apparatus depicted in Figure 1 and 2. From Figure 3 the contours 26 of the fan-shaped beam 2 in panoramic mode and the contours 27 of the fan-shaped beam 2 in cephalographic mode can be recognized. It can further be recognized that the beam 2 comprises an optical axis 28, which intersects an X-ray sensitive area 29 of the panoramic X-ray detector 6 in the panoramic operation mode and an X-ray sensitive area 30 of the cephalographic detector 18 in the cephalographic operation mode.

In the cephalographic operation, a projection of the head 16 is produced. This image is generally used for planning orthodontic measures. Therefore the projection should reflect the actual geometry of the head as accurate as possible.

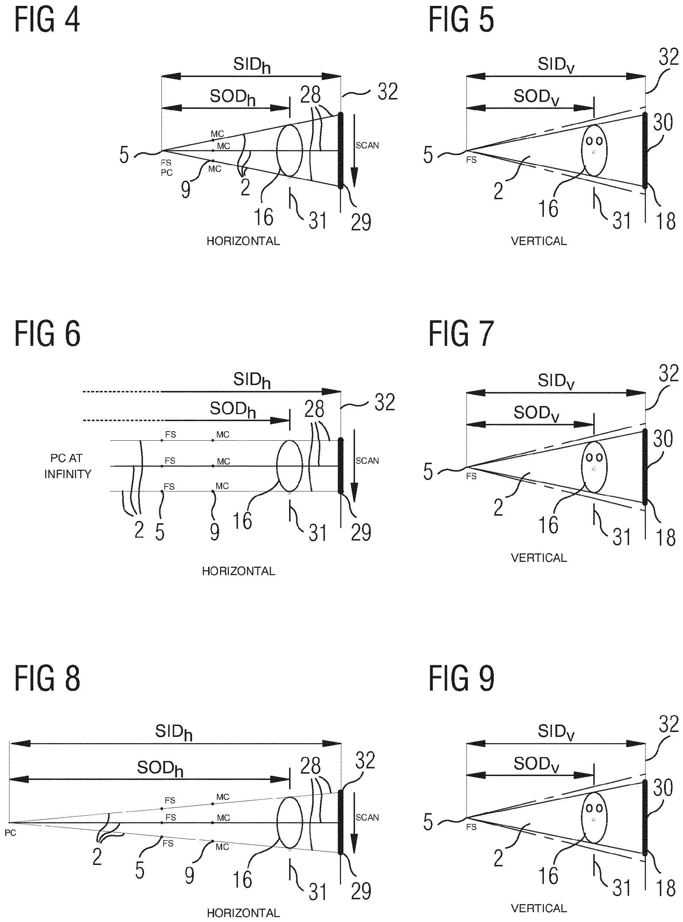

Figure 4 illustrates the projection geometry in a top view and Figure 5 illustrates the projection geometry in a side view. In the top view of Figure 4, the rays of the X-ray beam 2 are projected onto a horizontal plane. Figure 4 therefore illustrates the position of the optical axis 28 at different instants of time during the scanning process. The scanning in horizontal direction therefore results in an effective beam whose projection geometry can be recognized from the projection onto the horizontal plane. Figure 4 and 5 illustrate now the beam geometry of an embodiment, in which the rotation axis 9, which forms the mechanical center (= MC), is shifted during a scan in the horizontal direction in such a manner, that the projection center (= PC) in the horizontal plane coincides with the focal spot 5 (= FS) of the X-ray source 4. This can be achieved by shifting the rotation axis 9 along the horizontal axes 12 and 13 while the beam is pivoted around the rotation axis 9 for scanning the head 16 of the patient 15 in the horizontal direction.

By comparing Figure 4 and 5, it can easily be recognized that the embodiment of Figure 4 and 5 comprises the same vertical and horizontal magnification. The magnification of an object within an object plane 31 is determined by the ratio of the distance between source (= projection center) and detector 18 (= source-image-distance = SID) and the distance between source and object (= source-object-distance = SOD). Since SID and SOD are equal in the top view of Figure 4 and the side view of Figure 5, the horizontal magnification factor mₕ = SIDₕ/SODₕ equals the vertical magnification factor mᵥ = SIDᵥ/SODᵥ.

It should be noted that the distance between source and detector 18 is effectively the distance between source and the sensitive area 30 of the detector 18, because it is the sensitive are 30 of the detector 18, which defines an image plane 32.

The distance between source and the detector (SID) is determined by the geometry of the apparatus 1. But the distance between source and object (SOD) depends on the position of the object. Since the head 16 extends spatially along the optical axis 28, different parts of the head 16 are projected with a different magnification factors, resulting in a distortion of the projected image of the head 16.

Figure 6 and 7 illustrate now a configuration, in which the rotation axis 9, which forms the rotation center 9, is shifted in such a manner that the effective projection center (PC) is moved in the horizontal plane at infinity resulting in an effective beam with parallel ray geometry in the horizontal projection of Figure 6. In this case, the difference between SIDₕ and SODₕ becomes negligible resulting in a horizontal magnification factor mᵥ ≈ 1 independent from the position of the object plane 32 associated with a particular object. Thus, there is no distortion in the horizontal direction.

It should be noted that the distortion in the vertical direction is still present since the focal spot is still the projection center for the beam in the projection on a vertical plane. But what is more important is that there is no distortion in the horizontal direction, which is the important direction for orthodontic planning purposes.

The embodiment shown in Figure 6 and 7 requires that the rotation axis 9 can be shifted to such an extent that the entire head 16 can be scanned.

Figure 8 and 9 illustrate another embodiment, in which the cinematic capabilities for translating the rotation axis 9 along a horizontal plane are limited. In this case the virtual projection center can still be transferred along the optical axis 28 at a location behind the focal spot 5 (FS) resulting in a considerable reduced distortion of the object in horizontal direction.

The concept of locating a virtual projection center (PC) behind the focal spot 5 can also be used for panoramic imaging. In panoramic imaging, tomographic reconstruction methods are used for generating sectional views and volume images of the head 16, in particular the dentition of the patient 15. These methods require the acquisition of a series of two-dimensional images, which form a dataset. This dataset is then processed using well-known algorithms with the objective of reconstructing volume images and/or sectional views of the anatomic area under examination. The concept of locating the projection center (PC) at infinity, or at least locating the projection center (PC) as far as mechanically possible from the object, implies various advantages. The X-rays present in the incident X-ray beam diverge less, allowing a better three-dimensional tomographic reconstruction, in particular in the areas of the edge of the volume under examination (reduction of cone angle). Moreover, the projected image has a smaller magnification, and therefore, acquisition time and other parameters being equal, a bigger reconstructed volume is obtained. The increment is in the horizontal direction, which is the most important direction for orthodontic purposes.

The difference of magnification between the horizontal and the vertical direction can be easily corrected by rescaling the projected images. For this purpose, the same software algorithms can be used both for panoramic imaging and for tomographic reconstruction.

In the present description the X-ray beam 2 was described as having the shape of a fan extending along a vertically aligned plane. In practice, the X-ray beam 2 diverges also in the horizontal direction. In a modified embodiment, the detectors 6 and 18 may therefore also have more than a single column of detector pixels and thus may be also an area detector.

In another modified embodiment, the detector 6 may be stationary on the rotary arm 3 and the x-ray source 4 may perform a relative motion with respect to the rotary arm 3 to ensure that the panoramic detector 6 is out of the optical path of the X-ray beam 2 towards the cephalographic detector 18.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds or groups described in conjunction with a particular aspect, embodiment or example of the invention are to be understood to be applicable to any other aspect, embodiment or example described herein unless incompatible therewith.

## Claims

1. Apparatus for dental radiography (1) comprising:
- an imaging system comprising at least one X-ray detector (6, 18) having an X-ray sensitive area (29,30), the imaging system further comprising an X-ray source (4) having a focal spot (5, FS), which emits an X-ray beam (2) having an optical axis (28) which intersects with the X-ray sensitive area (29, 30) of the X-ray detector (6, 18),
- a support holding the imaging system and arranged for performing a trajectory of the imaging system for scanning a patient (15), wherein the trajectory of the imaging system comprises a pivotation of the X-ray source (4) around a mechanical rotation axis (MC) and a translation of the mechanical rotation axis (MC), wherein the combined pivotation and translation results in an effective pivotation of the X-ray source (4) around a virtual projection center (PC) of the X-ray beam (2), wherein the virtual projection center (PC) is located along the optical axis (28) behind the focal spot (5, FS) of the X-ray source (4) as seen from the X-ray detector (6, 18), **characterised in that**
the distance between the virtual projection center (PC) and the focal spot (5, FS) is greater than the distance between the focal spot (5, FS) and the detector (6, 18).

2. Apparatus according to Claim 1,
wherein the rotation axis (9) is aligned in the vertical direction and wherein the translation of the rotation axis (9) is performed along a horizontal plane.

3. Apparatus according to Claim 1 or 2,
wherein the at least one X-ray detector (6, 18) is a digital line detector and wherein the X-ray beam is fan-shaped.

4. Apparatus according to Claim 3,
wherein the longitudinal axis of the digital line detector (6, 18) is aligned in the vertical direction and wherein the fan-shaped X-ray beam extends along a vertical plane.

5. Apparatus according to Claim 1,
wherein the distance between the virtual projection center (PC) and the focal spot (5, FS) ranges between 3 meter and 5 meter.

6. Apparatus according to any one of Claim 1 to 5,
wherein the support comprises an extension arm (7) and a rotary arm (3) attached to the extension arm (7), which comprises a driving mechanism arranged for pivoting the rotary arm (3) around the rotation axis (9) and for translating the rotation axis (9), and wherein the X-ray source (4) is mounted on the rotary arm (3), and/or wherein the X-ray source (4) and/or the at least one X-ray detector (6, 18) can perform a relative motion with respect to the rotary arm (3).

7. Apparatus according to any one of Claim 1 to 6,
wherein the X-ray source (4) is mounted on one end of the rotary arm (3) and an X-ray detector (6) for panoramic imaging is mounted on the other end of the rotary arm (3), and/or wherein the support comprises a further extension (20) for holding a detector (18) for cephalography, wherein the detector (18) for cephalography is movable for keeping the detector (18) for cephalography in coincidence during the pivotation of the X-ray beam (2) around the rotation axis (9).

8. Apparatus according to Claim 6 or 7,
wherein a single detector (6, 18) is used for panoramic imaging and cephalography, the single detector (6, 18) being manually mountable on and detachable from the rotary arm (3) and on the further extension (20) for cephalography.

9. Apparatus according to any one of Claims 1 to 8,
wherein the apparatus comprises a control unit (14) arranged for controlling the trajectories of the imaging system in a panoramic operation mode and for controlling the trajectories of the imaging system in a cephalographic operation mode.

10. Method for dental radiography, wherein:
- an imaging system is used comprising at least one X-ray detector (6,18) having an X-ray sensitive area (29, 30), the imaging system further comprising an X-ray source (4) having a focal spot (5, FS), which emits an X-ray beam (2) having an optical axis (28) which intersects with the X-ray sensitive area (29,30) of the X-ray detector (6, 18),
- a support is used holding the imaging system, and
- a trajectory of the imaging system is performed for scanning a patient (15), wherein the trajectory of the imaging system comprises a pivotation of the X-ray source (4) around a mechanical rotation axis (MC and a translation of the mechanical rotation axis (MC), wherein the combined pivotation and translation results in an effective pivotation of the X-ray source (4) around a virtual projection center (PC) of the X-ray beam (2), wherein the virtual projection center (PC) is located along the optical axis (28) behind the focal spot (5, FS) of the X-ray source (4) as seen from the X-ray detector (6, 18) **characterised in that**
the distance between the virtual projection center (PC) and the focal spot (5, FS) is greater than the distance between the focal spot (5, FS) and the detector (6, 18).

11. Method according to Claim 10,
wherein the rotation axis (9) is aligned in the vertical direction and wherein the translation of the rotation axis (9) is performed along a horizontal plane.

12. Method according to Claim 10 or 11,
wherein the at least one X-ray detector (6, 18) is a digital line detector and wherein the X-ray beam (2) is fan-shaped.

13. Method according to Claim 12,
wherein the longitudinal axis of the digital line detector (6, 18) is aligned in the vertical direction and wherein the fan-shaped X-ray beam (2) extends along a vertical plane.

## Patentansprüche

1. Vorrichtung für die dentale Radiographie (1) mit:
- einem bildgebenden System, das wenigstens einen Röntgendetektor (6, 18) umfasst, der über einen für Röntgenstrahlen empfindlichen Bereich (29, 30) verfügt, wobei das bildgebende System ferner eine Röntgenquelle (4) mit einem Brennfleck (5, FS) verfügt, der einen Röntgenstrahl (2) mit einer optischen Achse (28) emittiert, die sich mit dem für Röntgenstrahlen empfindlichen Bereich (29, 30) des Röntgendetektors (6, 18) schneidet,
- einer Halterung, die das bildgebende System hält und die dazu eingerichtet ist, eine Bahn des bildgebenden Systems zum Abtasten eines Patienten (15) auszuführen, wobei die Bahn des bildgebenden Systems ein Verschwenken der Röntgenquelle (4) um eine mechanische Drehachse (MC) und eine Verschiebung der mechanischen Drehachse (MC) umfasst,
wobei die Kombination der Verschwenkung und der Verschiebung eine effektive Verschwenkung der Röntgenquelle (4) um ein virtuelles Projektionszentrum (PC) des Röntgenstrahls (2) bewirkt, wobei das virtuelle Projektionszentrum (PC) vom Röntgendetektor (6, 18) aus gesehen entlang der optischen Achse (28) hinter dem Brennfleck (5, FS) der Röntgenquelle (4) angeordnet ist,
**dadurch gekennzeichnet, dass**
der Abstand zwischen dem virtuellen Projektionszentrum (PC) und dem Brennfleck (5, FS) größer als der Abstand zwischen dem Brennfleck (5, FS) und dem Detektor (6, 18) ist.

2. Vorrichtung nach Anspruch 1,
bei der die Drehachse (9) in eine vertikale Richtung ausgerichtet ist, und bei der die Verschiebung der Drehachse (9) entlang einer horizontalen Ebene ausgeführt wird.

3. Vorrichtung nach Anspruch 1 oder 2,
bei der der wenigstens eine Röntgendetektor (6, 18) ein digitaler Liniendetektor ist und bei der der Röntgenstrahl fächerförmig ausgebildet ist.

4. Vorrichtung nach Anspruch 3,
bei der die Längsachse des digitalen Liniendetektors (6, 18) in eine vertikale Richtung ausgerichtet ist und bei der sich der fächerförmige Röntgenstrahl entlang einer senkrechten Ebene erstreckt.

5. Vorrichtung nach Anspruch 1,
bei der der Abstand zwischen dem virtuellen Projektionszentrum (PC) und dem Brennfleck (5, FS) zwischen 3 und 5 Metern liegt.

6. Vorrichtung nach einem der Ansprüche 1 bis 5,
bei der die Halterung einen Auslegerarm (7) und einen mit dem Auslegerarm (7) verbundenen Dreharm (3) umfasst, der einen Antriebsmechanismus aufweist, der dazu eingerichtet ist, den Dreharm (3) um die Drehachse (9) zu verschwenken und die Drehachse (9) zu verschieben, und bei der die Röntgenquelle (4) an dem Dreharm (3) angebracht ist und/oder bei der die Röntgenquelle (4) und/oder der wenigstens eine Röntgendetektor (6, 18) eine Relativbewegung bezüglich des Dreharms (3) ausführen kann.

7. Vorrichtung nach einem der Ansprüche 1 bis 6,
bei der die Röntgenquelle (4) an einem Ende des Dreharms (3) angebracht ist und bei der ein Röntgendetektor (6) für die Panoramabildgebung am anderen Ende des Dreharms (3) angebracht ist und/oder bei der die Halterung einen weiteren Ausleger (20) aufweist, um einen Detektor (18) für Cephalographie zu halten, wobei der Detektor (18) für Cephalographie beweglich ist, um den Detektor (18) für Cephalographie in Übereinstimmung mit der Verschwenkung des Röntgenstrahls (2) um die Drehachse (9) zu halten.

8. Vorrichtung nach Anspruch 6 oder 7,
bei der für die Panoramabildgebung und Cephalographie ein einzelner Detektor (6, 18) verwendet wird, wobei der einzelne Detektor (6, 18) von Hand an dem Dreharm (3) und an dem weiteren Ausleger (20) für Cephalographie anbringbar und von dort entfernbar ist.

9. Vorrichtung nach einem der Ansprüche 1 bis 8,
bei der die Vorrichtung eine Steuereinheit (14) aufweist, die dazu eingerichtet ist, die Bahnen des bildgebenden Systems in einem Panoramabetriebsmodus und die Bahnen des bildgebenden Systems in einem cephalographischen Betriebsmodus zu steuern.

10. Verfahren für die dentale Radiographie, bei dem:
- ein bildgebendes System verwendet wird, das wenigstens einen Röntgendetektor (6, 18) umfasst, der einen für Röntgenstrahlung empfindlichen Bereich (29, 30) hat, wobei das bildgebende System weiter eine Röntgenquelle (4) mit einem Brennfleck (5, FS) umfasst, der einen Röntgenstrahl (2) mit einer optischen Achse (28) emittiert, die sich mit dem für Röntgenstrahlen empfindlichen Bereich (29, 30) des Röntgendetektors (6, 18) schneidet,
- eine Halterung verwendet wird, die das bildgebende System hält, und
- eine Bahn des bildgebenden Systems zum Abtasten eines Patienten (15) ausgeführt wird, wobei die Bahn des bildgebenden Systems eine Verschwenkung der Röntgenquelle (4) um eine mechanische Drehachse (MC) und eine Verschiebung der mechanischen Drehachse (MC) umfasst,
wobei die Kombination der Verschwenkung und Verschiebung eine effektive Verschwenkung der Röntgenquelle (4) um ein virtuelles Projektionszentrum (PC) des Röntgenstrahls (2) bewirkt, wobei das virtuelle Projektionszentrum (PC) von dem Röntgendetektor (6, 18) aus gesehen entlang der optischen Achse (28) hinter dem Brennfleck (5, FS) der Röntgenquelle (4) liegt,
**dadurch gekennzeichnet, dass**
die Entfernung zwischen dem virtuellen Projektionszentrum (PC) und dem Brennfleck (5, FS) größer als die Entfernung zwischen dem Brennfleck (5, FS) und dem Detektor (6, 18) ist.

11. Verfahren nach Anspruch 10,
bei dem die Drehachse (9) in die vertikale Richtung ausgerichtet ist und bei dem die Verschiebung der Drehachse (9) entlang einer horizontalen Ebene erfolgt.

12. Verfahren nach Anspruch 10 oder 11,
bei dem der wenigstens eine Röntgendetektor (6, 18) ein digitaler Liniendetektor ist und bei dem der Röntgenstrahl (2) fächerförmig ist.

13. Verfahren nach Anspruch 12,
bei dem die Längsachse des digitalen Liniendetektors (6, 18) in eine senkrechte Richtung ausgerichtet ist, und bei dem der fächerförmige Röntgenstrahl (2) sich entlang einer senkrechten Ebene erstreckt.

## Revendications

1. Un appareil de radiographie dentaire (1) comprenant :
- un système d'imagerie comprenant au moins un détecteur à rayons X (6, 18) possédant une zone sensible aux rayons X (29, 30), le système d'imagerie comprenant en outre une source de rayons X (4) possédant un point focal (5, FS), qui émet un faisceau de rayons X (2) possédant un axe optique (28) qui vient en intersection avec la zone sensible aux rayons X (29, 30) du détecteur à rayons X (6, 18),
- un support maintenant le système d'imagerie et agencé de façon à exécuter une trajectoire du système d'imagerie de façon à balayer un patient (15), la trajectoire du système d'imagerie comprenant un pivotement de la source de rayons X (4) autour d'un axe de rotation mécanique (MC) et une translation de l'axe de rotation mécanique (MC),
dans lequel le pivotement et la translation combinés résultent en un pivotement effectif de la source de rayons X (4) autour d'un centre de projection virtuel (PC) du faisceau de rayons X (2), le centre de projection virtuel (PC) étant situé le long de l'axe optique (28) derrière le point focal (5, FS) de la source de rayons X (4) vu à partir du détecteur à rayons X (6, 18),
**caractérisé en ce que**
la distance entre le centre de projection virtuel (PC) et le point focal (5, FS) est supérieure à la distance entre le point focal (5, FS) et le détecteur (6, 18).

2. L'appareil selon la Revendication 1,
dans lequel l'axe de rotation (9) est aligné dans la direction verticale et dans lequel la translation de l'axe de rotation (9) est exécutée le long d'un plan horizontal.

3. L'appareil selon la Revendication 1 ou 2,
dans lequel le au moins un détecteur à rayons X (6, 18) est un détecteur de ligne numérique et dans lequel le faisceau de rayons X est en forme d'éventail.

4. L'appareil selon la Revendication 3,
dans lequel l'axe longitudinal du détecteur de ligne numérique (6, 18) est aligné dans la direction verticale et dans lequel le faisceau de rayons X en forme d'éventail s'étend le long d'un plan vertical.

5. L'appareil selon la Revendication 1,
dans lequel la distance entre le centre de projection virtuel (PC) et le point focal (5, FS) se situe dans la plage de 3 mètres à 5 mètres.

6. L'appareil selon l'une quelconque des Revendications 1 à 5,
dans lequel le support comprend un bras d'extension (7) et un bras rotatif (3) rattaché au bras d'extension (7), qui comprend un mécanisme d'entraînement agencé de façon à faire pivoter le bras rotatif (3) autour de l'axe de rotation (9) et à translater l'axe de rotation (9), et dans lequel la source de rayons X (4) est montée sur le bras rotatif (3), et/ou dans lequel la source de rayons X (4) et/ou le au moins un détecteur à rayons X (6, 18) peuvent exécuter un mouvement relatif par rapport au bras rotatif (3).

7. L'appareil selon l'une quelconque des Revendications 1 à 6,
dans lequel la source de rayons X (4) est montée sur une extrémité du bras rotatif (3) et un détecteur à rayons X (6) destiné à une imagerie panoramique est monté sur l'autre extrémité du bras rotatif (3), et/ou dans lequel le support comprend une autre extension (20) destinée à recevoir un détecteur (18) destiné à une céphalographie, le détecteur (18) destiné à une céphalographie étant déplaçable de façon à maintenir le détecteur (18) destiné à une céphalographie en coïncidence au cours du pivotement du faisceau de rayons X (2) autour de l'axe de rotation (9).

8. L'appareil selon la Revendication 6 ou 7,
dans lequel un détecteur unique (6, 18) est utilisé pour une imagerie panoramique et une céphalographie, le détecteur unique (6, 18) pouvant être monté manuellement sur et être détaché du bras rotatif (3) et monté sur l'autre extension (20) pour une céphalographie.

9. L'appareil selon l'une quelconque des Revendications 1 à 8,
dans lequel l'appareil comprend une unité de commande (14) agencée de façon à commander les trajectoires du système d'imagerie dans un mode de fonctionnement panoramique et à commander les trajectoires du système d'imagerie dans un mode de fonctionnement céphalographique.

10. Un procédé de radiographie dentaire, dans lequel :
- un système d'imagerie est utilisé comprenant au moins un détecteur à rayons X (6, 18) possédant une zone sensible aux rayons X (29, 30), le système d'imagerie comprenant en outre une source de rayons X (4) possédant un point focal (5, FS) qui émet an faisceau de rayons X (2) possédant un axe optique (28) qui vient en intersection avec la zone sensible aux rayons X (29, 30) du détecteur à rayons X (6, 18),
- un support est utilisé de façon à maintenir le système d'imagerie, et
- une trajectoire du système d'imagerie est exécutée de façon à balayer un patient (15), la trajectoire du système d'imagerie comprenant un pivotement de la source de rayons X (4) autour d'un axe de rotation mécanique (MC) et une translation de l'axe de rotation mécanique (MC),
dans lequel le pivotement et la translation combinés résultent en un pivotement effectif de la source de rayons X (4) autour d'un centre de projection virtuel (PC) du faisceau de rayons X (2), le centre de projection virtuel (PC) étant situé le long de l'axe optique (28) derrière le point focal (5, FS) de la source de rayons X (4) vu à partir du détecteur à rayons X (6, 18)
**caractérisé en ce que**
la distance entre le centre de projection virtuel (PC) et le point focal (5, FS) est supérieure à la distance entre le point focal (5, FS) et le détecteur (6, 18).

11. Le procédé selon la Revendication 10,
dans lequel l'axe de rotation (9) est aligné dans la direction verticale et dans lequel la translation de l'axe de rotation (9) est exécutée le long d'un plan horizontal.

12. Le procédé selon la Revendication 10 ou 11,
dans lequel le au moins un détecteur à rayons X (6, 18) est un détecteur de ligne numérique et dans lequel le faisceau de rayons X (2) est en forme d'éventail.

13. Le procédé selon la Revendication 12,
dans lequel l'axe longitudinal du détecteur de ligne numérique (6, 18) est aligné dans la direction verticale et dans lequel le faisceau de rayons X en forme d'éventail (2) s'étend le long d'un plan vertical.
